(19) **Europäisches Patentamt / European Patent Office / Office européen des brevets**

(11) **EP 3 773 677 B1**

(12) # EUROPEAN PATENT SPECIFICATION

(45) Date of publication and mention
of the grant of the patent:
**02.04.2025 Bulletin 2025/14**

(21) Application number: **19723883.5**

(22) Date of filing: **11.04.2019**

(51) International Patent Classification (IPC):
*A61K 38/18* (2006.01)     *A61P 25/28* (2006.01)
*A61P 19/10* (2006.01)     *A61P 9/10* (2006.01)

(52) Cooperative Patent Classification (CPC):
**A61K 38/1866; A61P 9/10; A61P 19/10;
A61P 25/28**

(86) International application number:
**PCT/IL2019/050409**

(87) International publication number:
**WO 2019/198084 (17.10.2019 Gazette 2019/42)**

(54) **ANTI-AGING COMPOSITIONS AND METHODS OF USE**

ANTI-AGING-ZUSAMMENSETZUNGEN UND VERFAHREN ZUR VERWENDUNG

COMPOSITIONS ANTI-ÂGE ET PROCÉDÉS D'UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.04.2018 US 201862656471 P**

(43) Date of publication of application:
**17.02.2021 Bulletin 2021/07**

(73) Proprietor: **Yissum Research Development
Company of the Hebrew
University of Jerusalem Ltd.
91390 Jerusalem (IL)**

(72) Inventors:
• **GRUNEWALD, Miriam
9094200 Elazar (IL)**
• **KESHET, Eli
9088500 Aminadav (IL)**

(74) Representative: **Boult Wade Tennant LLP
Salisbury Square House
8 Salisbury Square
London EC4Y 8AP (GB)**

(56) References cited:
**EP-A1- 2 714 063        EP-A1- 3 011 957
WO-A1-2014/020149    WO-A1-2016/092453**

WO-A2-01/76620        WO-A2-2007/007362
CN-A- 107 661 492

• JIN BO TANG ET AL: "Basic FGF or VEGF gene
therapy corrects insufficiency in the intrinsic
healing capacity of tendons", SCIENTIFIC
REPORTS, vol. 6, no. 1, 11 February 2016
(2016-02-11), pages 1 - 12, XP055543950, DOI:
10.1038/srep20643
• BRIDGET M DEASY ET AL: "Effect of VEGF on
the Regenerative Capacity of Muscle Stem Cells
in Dystrophic Skeletal Muscle", MOLECULAR
THERAPY, vol. 17, no. 10, 1 October 2009
(2009-10-01), pages 1788 - 1798, XP055030468,
ISSN: 1525-0016, DOI: 10.1038/mt.2009.136
• AZZOUZ MIMOUN ET AL: "VEGF delivery with
retrogradely transported lentivector prolongs
survival in a mouse ALS model", NATURE,, vol.
429, no. 6990, 27 May 2004 (2004-05-27), pages
413 - 417, XP002471751, DOI: 10.1038/
NATURE02544

EP 3 773 677 B1

- ROGERS R S ET AL: "Intracavernosal vascular endothelial growth factor (VEGF) injection and adeno-associated virus-mediated VEGF gene therapy prevent and reverse venogenic erectile dysfunction in rats", INTERNATIONAL JOURNAL OF IMPOTENCE RESEARCH, STOCKTON, BASINGSTOKE, GB, vol. 15, no. 1, 1 February 2003 (2003-02-01), pages 26 - 37, XP037777572, ISSN: 0955-9930, [retrieved on 20030226], DOI: 10.1038/SJ.IJIR.3900943

- HILTUNEN MIKKO OLAVI ET AL: "Adenovirus-mediated VEGF-A gene transfer induces bone formation in vivo", THE FASEB JOURNAL, FEDERATION OF AMERICAN SOCIETIES FOR EXPERIMENTAL BIOLOGY, US, vol. 17, no. 9, 1 June 2003 (2003-06-01), pages 1 - 15, XP002539332, ISSN: 0892-6638, [retrieved on 20030408], DOI: 10.1096/FJ.02-0514FJE

**Description**

CROSS-REFERENCE TO RELATED APPLICATIONS

This application claims the benefit of priority of U.S. Provisional Patent Application No. 62/656,471 titled "ANTI-AGING COMPOSITIONS AND METHODS OF USE", filed April 12, 2018.

FIELD OF INVENTION

The present invention is in the field of anti-aging pharmacology

BACKGROUND OF INVENTION

Aging can be defined as an inevitable, irreversible decline in organ function that occurs over time even in the absence of injury, illness, environmental risks, or poor lifestyle choices (e.g., unhealthy diet, lack of exercise, substance abuse). Initially, the changes in organ function do not affect baseline function; the first manifestations are a reduced capacity of each organ to maintain homeostasis under stress (e.g., illness, injury). The cardiovascular, renal, and central nervous systems are usually the most vulnerable.

Various diseases interact with pure aging effects to cause geriatric-specific complications, particularly in the cardiovascular, renal, and central nervous systems, even when those organs are not the primary ones affected by a disease. Typical examples are delirium complicating pneumonia or urinary tract infection (UTI) and the falls, dizziness, syncope, urinary incontinence, and weight loss that often accompany many minor illnesses in the elderly. Aging organs are also more susceptible to injury; e.g., intracranial hemorrhage is more common and is triggered by less clinically important injury in the elderly.

Patent application no. CN107661492 relates to applications of using VEGF-B for the treatment of uncomfortable diseases as caused by oxidative stress damage.

Patent application no. WO 2001/76620 relates to use of VEGF for treating neurological and physiological dysfunction associated with neuron disorders.

Patent application no. EP 3011957 relates to a composition for use in stimulating vascularization in a subject, the composition comprising dapsone or N-acetyl-dapsone.

Patent application no. WO 2016/092453 relates to prevention, treatment and slowing the progression of brain pathology in a disease or condition associated with ApoE4 genotype by activation of VEGF in the hippocampus of a subject.

Patent application no. EP 2714063 relates to methods for modulating the length and/or the complexity of the dendrites of a neuronal cell by influencing the amount of vascular endothelial growth factor D (VEGF-D)-related signaling.

Patent application no. WO 2007/007362 relates to the use of VEGF-D or fragments thereof for the preparation of a medicament for therapy and / or prevention of bone diseases.

Patent application no. WO 2014020149 relates to adeno-associated viral vector useful for transducing adipose tissue.

Additional publications include:

Jin Bo Tang ET AL: "Basic FGF or VEGF gene therapy corrects insufficiency in the intrinsic healing capacity of tendons", Scientific Reports, vol. 6, no. 1, 11 February 2016 (2016-02-11), pages 1-12, XP055543950, DOI: 10.1038/srep20643;

Bridget M Deasy ET AL: "Effect of VEGF on the Regenerative Capacity of Muscle Stem Cells in Dystrophic Skeletal Muscle", Molecular Therapy, vol. 17, no. 10, 1 October 2009 (2009-10-01), pages 1788-1798, XP055030468, ISSN: 1525-0016, DOI: 10.1038/mt.2009.136;

AZZOUZ MIMOUN ET AL: "VEGF delivery with retrogradely transported lentivector prolongs survival in a mouse ALS model", NATURE, vol. 429, no. 6990, 27 May 2004 (2004-05-27), pages 413-417, XP002471751, DOI: 10.1038/NATURE02544;

ROGERS R S ET AL: "Intracavernosal vascular endothelial growth factor (VEGF) injection and adeno-associated virus-mediated VEGF gene therapy prevent and reverse venogenic erectile dysfunction in rats", INTERNATIONAL JOURNAL OF IMPOTENCE RESEARCH, STOCKTON, BASINGSTOKE, GB, vol. 15, no. 1, 1 February 2003 (2003-02-01), pages 26-37, XP037777572, ISSN: 0955-9930, DOI: 10.1038/SJ.IJIR.3900943; and

HILTUNEN MIKKO OLAVI ET AL: "Adenovirus-mediated VEGF-A gene transfer induces bone formation in vivo", THE FASEB JOURNAL, FEDERATION OF AMERICAN SOCIETIES FOR EXPERIMENTAL BIOLOGY, US, vol. 17, no. 9, 1 June 2003 (2003-06-01), pages 1-15, XP002539332, ISSN: 0892-6638, DOI: 10.1096/FJ.02-0514FJE.

As the average life span increases in developed countries, which is primarily attributed to medical breakthroughs and improvements in nutrition and lifestyle, treatments that can slow aging or treat aging-related disorders are greatly in need.

SUMMARY OF THE INVENTION

The present invention is directed to a pharmaceutical composition comprising a vascular endothelial growth factor (VEGF)-stimulating compound for use in a method of treating age-related symptoms or disease selected from whole body weight loss of the elderly, kyphosis, pancreatic steatosis and hepatosteatosis, wherein the VEGF-stimulating compound is a nucleic acid comprising a VEGF-A encoding sequence, wherein the method comprises administering a therapeutically effective amount of the pharmaceutical composition to a subject in need thereof.

In some preferred embodiments, the pharmaceutical composition for use according to the invention is administered in an amount effective to increase the amount of free or circulating VEGF-A by 3-fold at most in a subject's plasma compared to a baseline, wherein the baseline is basal VEGF-A plasma levels in the subject.

In some preferred embodiments, the 3-fold increased amount of free or circulating VEGF-A compared to the baseline is maintained for at least 30 days.

In some preferred embodiments, the pharmaceutical composition for use according to the invention is administered at least once a month. In some preferred embodiments, the pharmaceutical composition for use according to the invention is administered once a month.

Unless otherwise defined, all technical and/or scientific terms used herein have the same meaning as commonly understood by one of ordinary skill in the art to which the invention pertains.

Further embodiments and the full scope of applicability of the present invention will become apparent from the detailed description given hereinafter.

BRIEF DESCRIPTION OF THE DRAWINGS

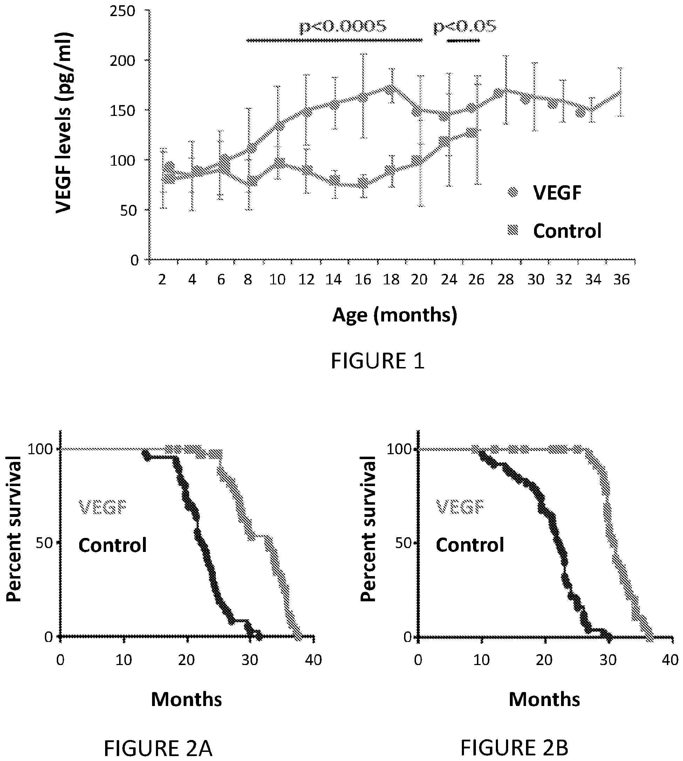

**Fig. 1** is a graph describing VEGF plasma levels in mice. Control individuals (Control) had basal low VEGF plasma levels, that were significantly lower (p<0.0005) compared to individuals over-expressing VEGF (VEGF), from the 8th month onwards. A gradual increase in control individuals was observed from month 20 onwards. Measurements of VEGF levels in the plasma had ceased on month 26 in the control group due to 100% mortality or poor health conditions, while persisted for the VEGF over-expressing animals until month 36.

**Figs. 2A-2B** are graphs describing survival proportion among males (**2A**) and females (**2B**) mice. Individuals over-expressing VEGF (VEGF) outlived control (Control). All control mice died by month 31.5 (males; **2A**) and by month 30 (females; **2B**), during which survival was 52% (males; **2A**) and 59% (females; **2B**) for VEGF mice. VEGF mice maximum lifespan was extended to 37.6 months (males; **2A**) and 36.46 months (females; **2B**). The median survival for control mice was 22.7 months (males; **2A**) and 22.2 months (females; **2B**), whereas it was 33 months for VEGF male mice and 30.85 months for VEGF female mice. Differences in the survival curves were found to be statistically significant with a P value lower than 0.0001 (Gehan-Breslow-Wilcoxon Test). A month was considered to have an average of 30.42 days.

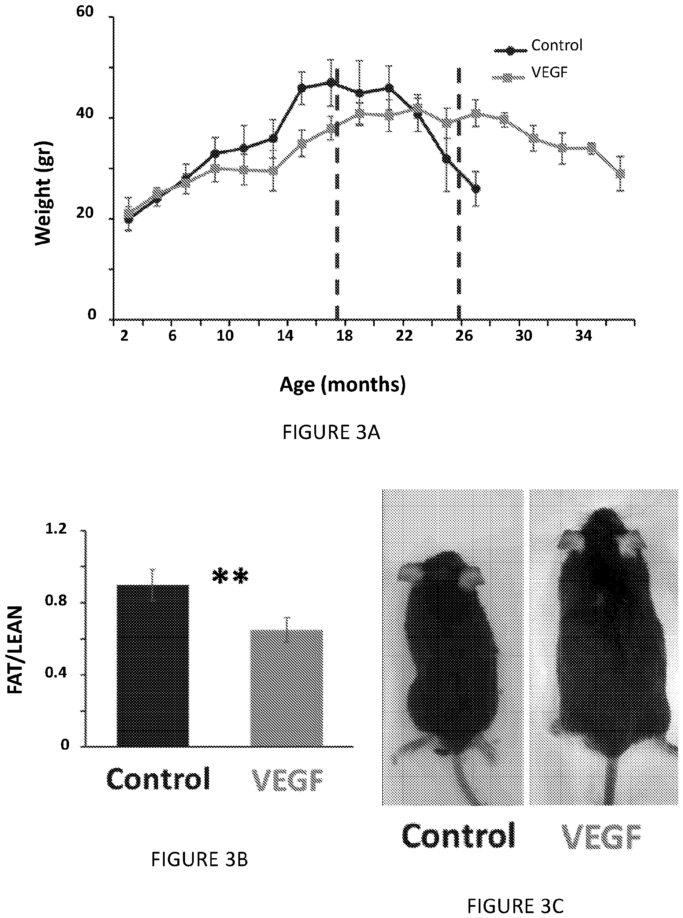

**Figs. 3A-3C** are graphs and images describing weight gain of male mice. (**3A**) Starting from 12 months of age, VEGF male mice gained significantly less weight than their control littermates. Body composition analysis by Echo-MRI showed less fat accumulation in VEGF mice at 16 months of age compared to their control littermates (**3B**). VEGF mice maintain their weight throughout their adult life while a significant weight loss was observed in aged control mice (**3B**). At day of sacrifice, control individuals were significantly more cachectic than their VEGF littermates, weighing 27 ± 3 gr and 40 ± 5 gr, respectively. (**3C**) overview image of 24 months old control and VEGF mice.

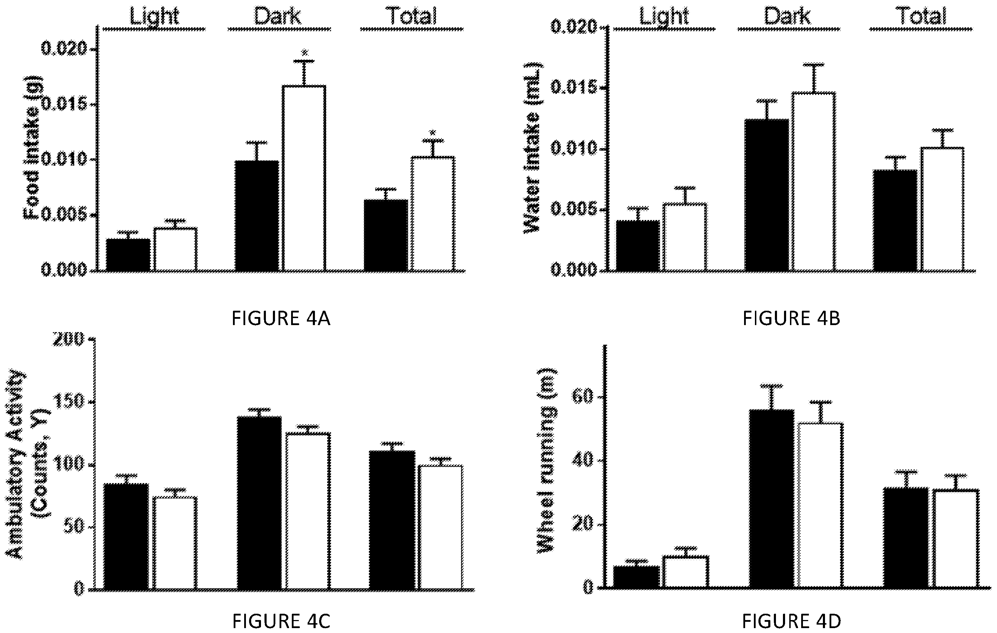

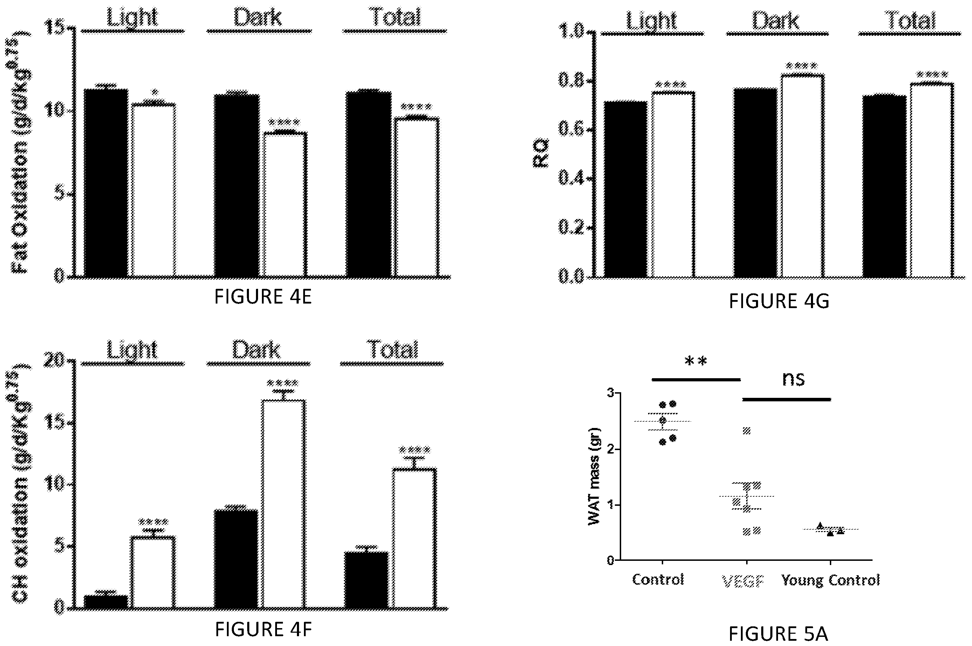

**Figs. 4A-4G** are vertical bar graphs comparing various behavior and metabolic parameters between control (white bars) and VEGF (black bars) mice. VEGF mice were found to have significantly increased (females) or similar (males, not shown) food intake (**4A**), and similar water intake (**4B**). Both control and VEGF mice were found to have similar ambulatory (**4C**) and voluntary activities (**4D**). (**4E-4F**) are graphs describing the relative amount of fat (**4E**) and carbohydrate (**4F**) oxidation (based on measurements of oxygen consumption and $CO_2$ exhalation) demonstrating that VEGF mice conserved a higher metabolic flexibility while aging also reflected by **Fig 4G**. (**4G**) is a graph showing the respiratory quotient (RQ). RQ was calculated from the ratio of carbon dioxide produced by the body of the mouse to oxygen consumed by the body, indicating which macronutrients were being metabolized (0.7 for solely fat, 0.8 for solely proteins, 1 for solely carbohydrates).

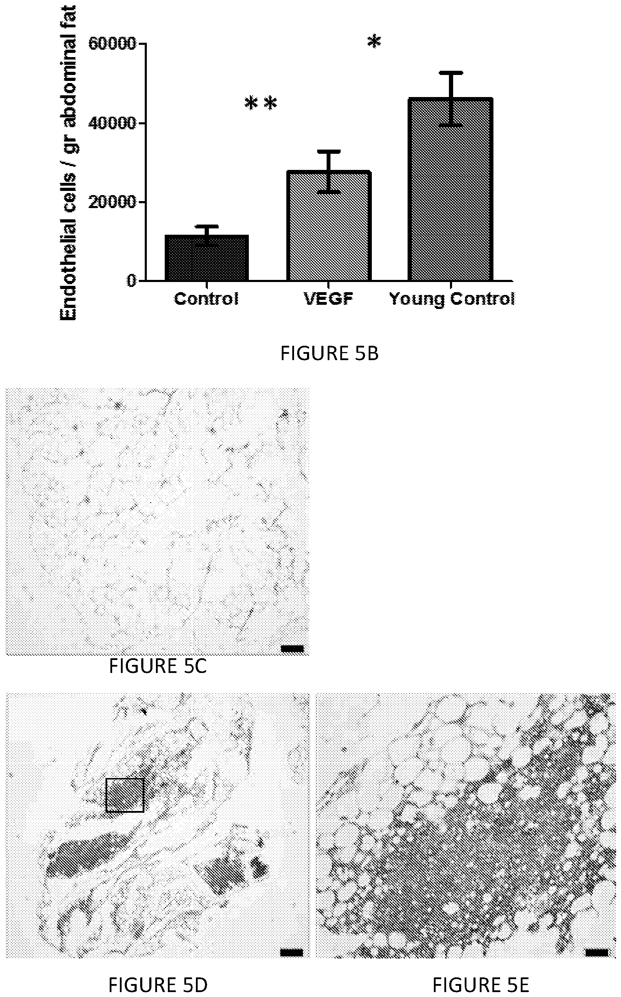

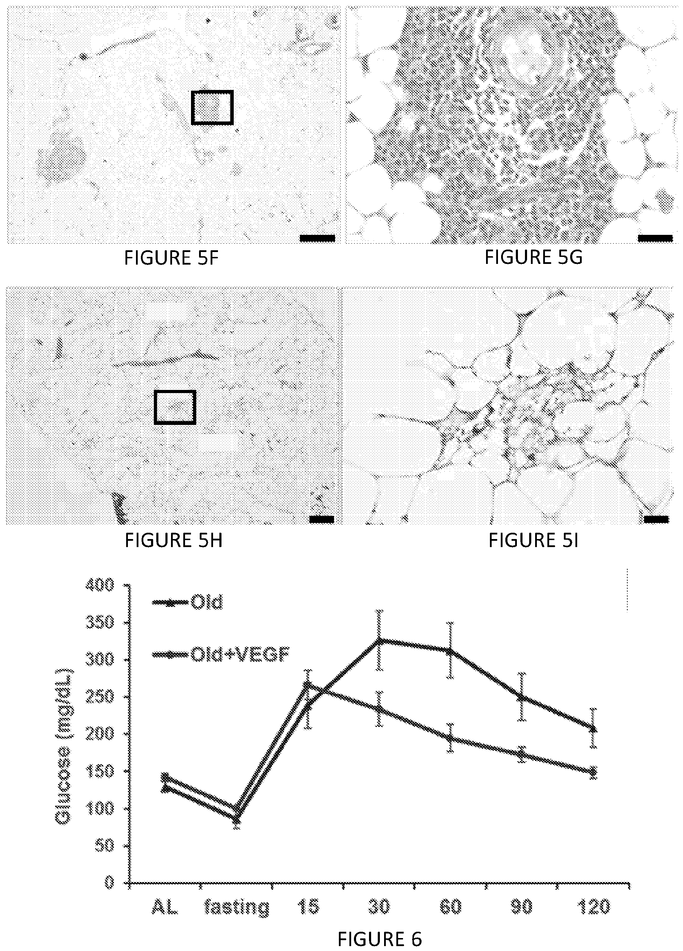

**Figs. 5A-5I** are graphs and micrographs demonstrating comparative dynamics in white adipose tissue (WAT) weight and activity. At 18 months, a significant reduction in WAT mass was observed in VEGF mice compared to their control littermates (**5A**), accompanied by adequate perfusion maintenance of this tissue (as reflected by the number of endothelial cells per gram tissue; **5B**). (**5C-5E**) are micrographs of histological sections of abdominal WAT isolated from control (**5C**) and VEGF-overexpressing mice (**5D-5E**). Specific staining for uncoupling protein 1 (UCP1) highlighted islands of beige adipocytes, which are known to have high thermogenic capacity, only in the VEGF mice (**5D**). (**5E**) is an enlarged area defined by a square in **5D**. In addition, control WAT was found to accommodate larger immune cell infiltrates (**5F-5G**), compared to WAT obtained from VEGF (**5H-5I**). (**5G**) **and** (**5I**) are enlarged areas defined by squares in **5F** and **5H**, respectively. Scale bar: **5C**-5H = 200 μm; **5E** = 50 μm; **5G** and **5I** = 20 μm.

**Fig. 6** is a graph showing a better glucose tolerance in 18 months old VEGF male mice compared to their control littermates. AL - ad libitum.

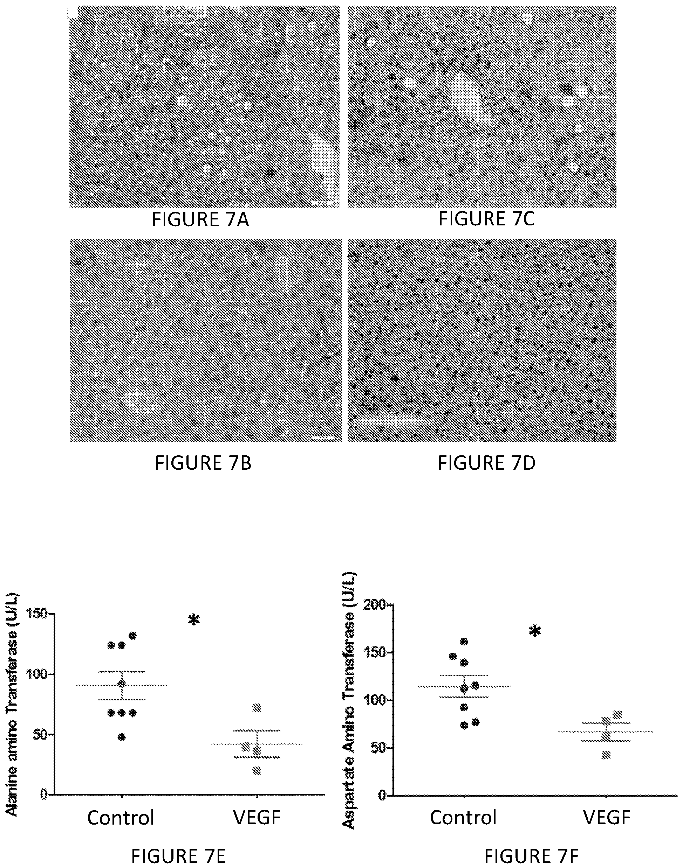

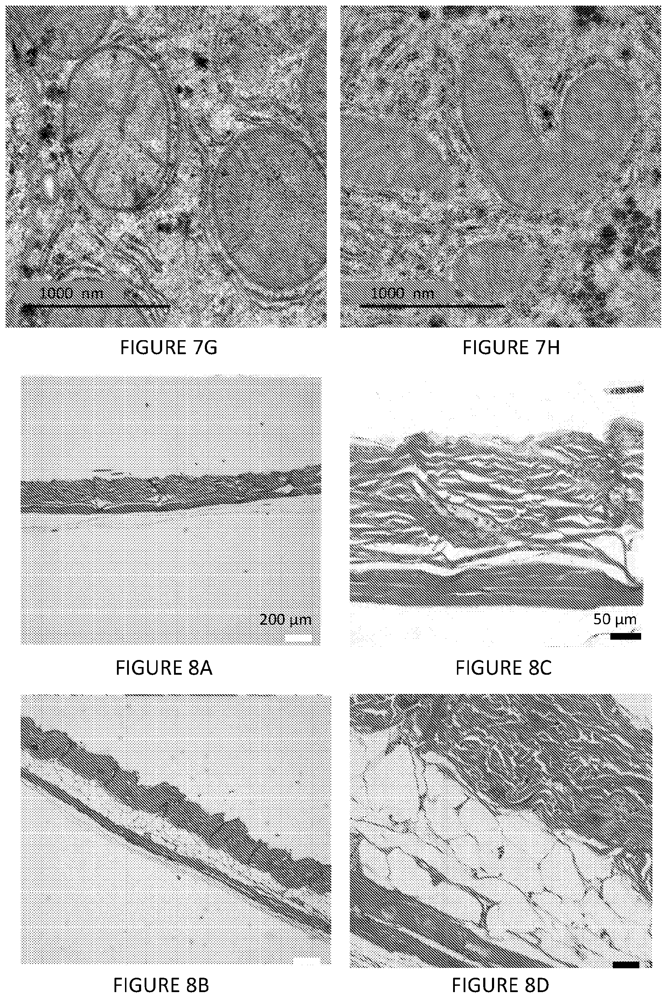

**Figs. 7A-7H** are pictures and graphs showing differential liver structurality and functionality for control and VEGF mice. At 24 months of age, livers of control mice presented advanced steatosis (**7A**) characterized by lipid accumulation (evidenced by Oil-red O staining as large gray droplets; **7C**) as well hepatocytes' injury, as demonstrated by increased serum concentrations of ALT (**7E**) and AST (**7F**). In contrast, VEGF mice were found to be protected from aging-associated hepatic steatosis and hepatocytes injury (**7C-7F**). Such lipid accumulations were not observed in VEGF-overexpressing mice (**7B and 7D**). Bars = 50 $\mu$m. (**7G-7H**) are high magnification electron microscopy images of hepatocytes from livers of control and VEGF mice. Mitochondria observed in control hepatocytes were enlarged and displayed disorganized cristae (**7G**). The rough endoplasmic reticulum (RER) appeared to be swollen and sparsely decorated with ribosomes which indicated ATP depletion induced-stress in control hepatocytes. In contrast, mitochondria and RER in VEGF mice were shown to have a normal appearance (**7H**). Scale bar = 1,000 nm in **7G-7H**.

**Figs. 8A-8D** are micrographs of histological sections of dorsal skin tissues obtained from 24 months old control (**8A**-**8B**) and VEGF over-expressing (**8C**-**8D**) mice, which were stained with Masson's trichrome stain. A very few adipocytes were observed in sections of control mice skin (**8A**) reflecting a case of major hypodermal fat loss. In contrast, VEGF over-expressing mice, demonstrated a rich layer of adipose tissue (**8B and 8D**). **8C** and **8D** are enlargements of areas from **8A** and **8B**, respectively. Scale bar: (**8A-8B**) = 200 $\mu$m; (**8C-8D**) = 50 $\mu$m.

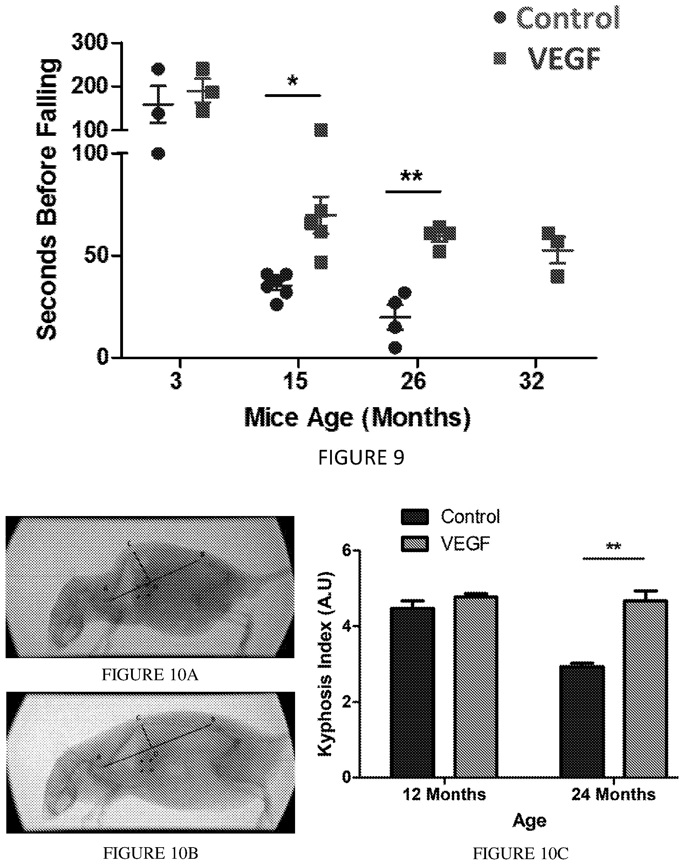

**Fig. 9** is a graph describing performance of mice following a ROTAROD test that evaluates balance, grip strength and motor coordination and is measured as seconds before falling. At 15 months of age and onward, VEGF mice (■) showed better performance than their control littermates (•).

**Fig. 10A-10C** are images and a graph demonstrating differential kyphosis index (KI) for control and VEGF mice. (**10A-10B**) are representative high resolution X-ray radiographs of control (**10A**) and VEGF (**10B**) 24 months old mice which were sedated (50 mg/kg ketamine and 10 mg/kg xylazine HCl administered by s.c.i.), lightly taped to the table support, radiographed (52 kVp, 4.30 mA) and had their KI calculated. At 24 months of age, the KI of control mice was found to be significantly lower than that of the VEGF-overexpressing mice (by approximately 1.5-fold; **10C**). Further, VEGF mice were shown to maintain their KI when comparing it at the ages of 12 months and 24 months (**10C**). AB - connecting line drawn from posterior edge of C7 (A) to posterior edge of L6 (B); CD - connecting line drawn from dorsal border of vertebral body farthest from AB line; KI=AB/CD. Lower KI indicates more severe kyphosis.

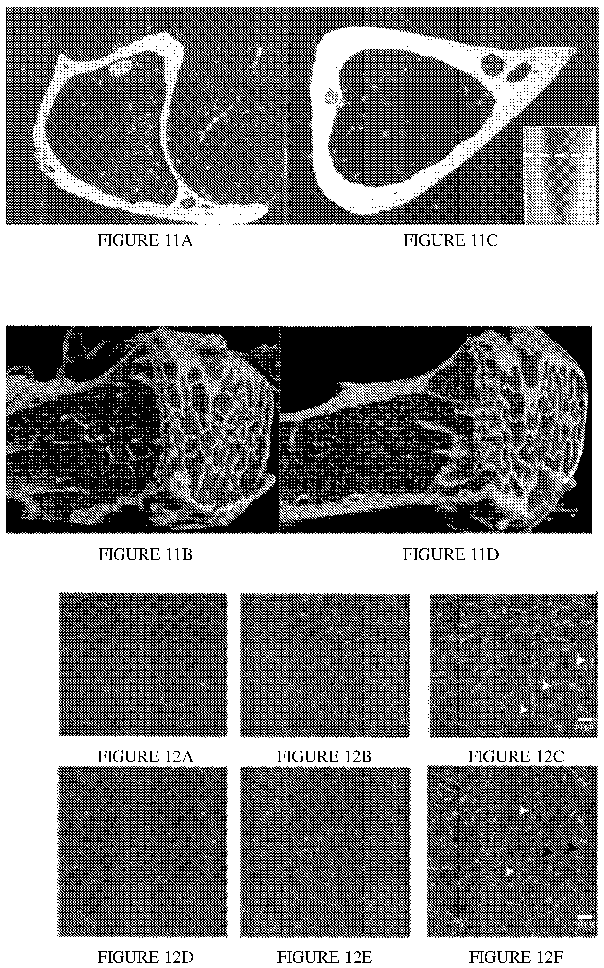

**Figs. 11A-11D** are high resolution micro computational tomography (CT) images of cross sections (see inset in **11C** for section orientation) through femoral bones of control (**11A-11B**) and VEGF-overexpressing mice (**11C-11D**). Bone morphometry was assessed by measuring bone to tissue volume ratio. In average, control mice lose 22% more bone tissue than their VEGF littermates.

**Figs. 12A-12F** are fluorescent images of bone tissue. Bones were dissected from control **A, B** and **C**) and VEGF over-expressing (VEGF; **D, E** and **F**) 15 months old mice, decalcified, sectioned and immunostained with the endothelial markers Endomucin (**12A and 12D**) and CD31 (**12B and 12E**). CD31$^+$EMCN$^{NEG}$ elongated (white arrow heads) and branched (black arrow heads) arteries were observed in the diaphysis of VEGF over-expressing mice (**12F**). Imaging was done using a confocal laser-scanning microscopy, using the z-stack scanning to obtain sequential depth imaging of thick bone sections. Three dimensional (3D) reconstructions of images were done using Imaris software. Bar = 50 $\mu$m.

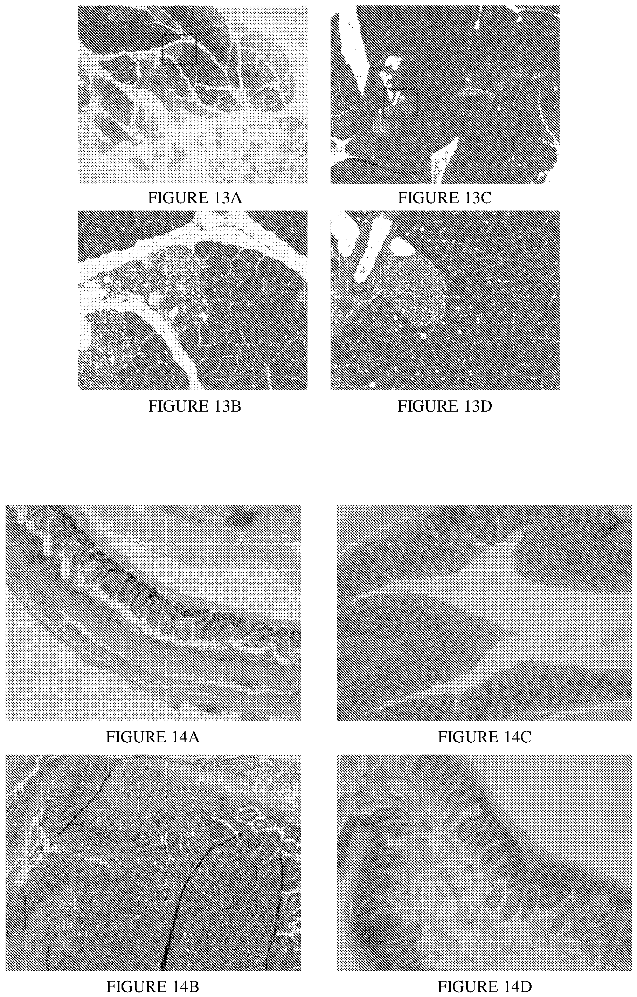

**Figs.** 13A-13D are micrographs of the pancreas. Pancreatic tissues were dissected from control (**13A-13B**) and VEGF over-expressing (**13C-13D**) 24 months old mice, fixed in formalin and processed for paraffin embedding. Sections of 6 $\mu$m were stained with standard Hematoxylin and Eosin stain. In sections of control mice, a substantial pancreatic steatosis was observed in numerous lobes and lobules (**13A-13B**). In contrast, sections of VEGF over-expressing mice demonstrated hallmark features of a healthy pancreas, as demonstrated by packed lobes and lobules comprising both endocrine islets and exocrine acini (**13C-13D**). (**13B**) and (**13D**) represent enlargements of the regions defined in squares in (**13A**) and (**13C**), respectively.

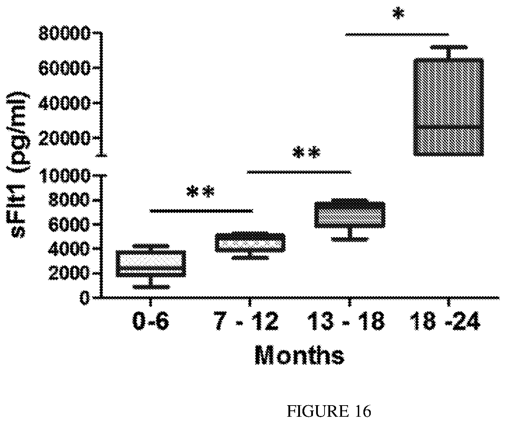

**Figs. 14A-14D** are images of histological sections of colon (**14A** and **14C**) and duodenum (**14B** and **14D**) tissues, from control (**14A-14B**) and VEGF over expressing (**14C-14D**) mice. Intestine tissues were dissected from 24 months old mice, fixed in formalin and processed for paraffin embedding. Sections of 6 $\mu$m were stained with standard Hematoxylin and Eosin stain. In sections of control mice colon, villi were found to be short and aged in appearance, starved, uniformly spaced and oddly shaped with large immune aggregates, undigested food particles and disordered villi were also observed (**14A**). Sections of the duodenum revealed fat deposition, extensive adenomas, undigested food particles and the presence of numerous early polyps (**14B**). In contrast, colon and duodenum villi in VEGF-overexpressing mice were completely normal in appearance with no documentation of adenoma. Nevertheless, some early polyps were observed (**14C-14D**). **Figs**. **15A**-**15B** are graphs describing decrease in cancer-related phenomena in VEGF-overexpressing mice compared to control. (**15A**) is a graph showing the % of mice which presented at least one spontaneous tumor type at time of sacrifice. In both female and male control mice, neoplastic lesions were observed more often than in their VEGF littermates. (**15B**) is a graph showing a significant increase in circulating granulocytes in the blood of control mice as compared to their VEGF littermates. **Fig. 16** is a graph describing the increase in circulating soluble VEGF Receptor 1 (VEGFR1, i.e., sFlt1) in aging control mice. During the last months of life, control aged mice comprised significantly higher sFlt1 levels compared to control young mice. Each group age comprised more than 8 mice.

DETAILED DESCRIPTION OF THE INVENTION

The present invention relates to a pharmaceutical composition comprising a vascular endothelial growth factor (VEGF)-stimulating compound for use in a method of treating age-related symptoms or disease selected from whole body weight loss of the elderly, kyphosis, pancreatic steatosis and hepatosteatosis, wherein the VEGF-stimulating compound is a nucleic acid comprising a VEGF-A encoding sequence, wherein the method comprises administering a therapeutically effective amount of the pharmaceutical composition to a subject in need thereof.

The present invention is based, in part, on the finding that controlled elevation of VEGF plasma levels attenuated symptoms associated with age-related symptoms and diseases in mice. Specifically, mildly increased VEGF signaling reduced muscle wasting and muscle mass loss, reduced pancreatic and hepatic steatosis, reduced subdermal fat loss, reduced weight gain during adulthood, induced and maintained brown adipose tissue, reduced age-related bone loss and fragility and attenuated age-related whole-body weight loss. Simultaneously, improved motor and coordination activity and prolonged life span were observed. The invention is further based, in part, on the finding that artificial mild increase of VEGF signaling by up to 3-fold, a therapeutic anti-aging response relevant for age-related symptoms and diseases can be achieved.

In some preferred embodiments, the pharmaceutical composition for use according to the invention is administered in an amount effective to increase the amount of free or circulating VEGF-A by 3-fold at most in a subject's plasma, as compared to a baseline wherein the baseline is basal VEGF-A plasma levels in the subject. In some preferred embodiments, the 3-fold increased amount of free or circulating VEGF-A compared to the baseline is maintained for at least 30 days.

In one preferred embodiment, "age-related" addresses a subject older than 50 years of age. In another preferred embodiment, "age-related" addresses a subject older than 60 years of age. In another preferred embodiment, "age-related" addresses a subject older than 70 years of age. In another preferred embodiment, "age-related" addresses a subject older than 75 years of age. In another preferred embodiment, "age-related" addresses a subject older than 80 years of age. In another preferred embodiment, "age-related" addresses a subject older than 85 years of age. In another preferred embodiment, "age-related" addresses a subject older than 90 years of age. In another preferred embodiment, "age-related" addresses a subject older than 95 years of age. In another preferred embodiment, "age-related" addresses a subject older than 99 years of age.

In one preferred embodiment, "age-related" addresses a subject older than 50-70 years of age, 60-80 years of age, 75-95 years of age, or 85-99 years of age. Each possibility represents a separate preferred embodiment of the invention.

As used herein, the terms "age-related" and "age-associated" are interchangeable.

In some preferred embodiments, the pharmaceutical composition for use according to the invention is for use in a method for treating whole body weight loss of the elderly. As defined herein, "weight loss" refers to the reduction of total body mass in a subject. In one preferred embodiment, weight loss is a reduction of at least 3% of whole-body mass in a subject. In another preferred embodiment, weight loss is a reduction of at least 4% of whole-body mass in a subject. In another preferred embodiment, weight loss is a reduction of at least 5% of whole-body mass in a subject. In another preferred embodiment, weight loss is a reduction of at least 10% of whole-body mass in a subject. In another preferred embodiment, weight loss is a reduction of at least 20% of whole-body mass in a subject. In another preferred embodiment, weight loss is a reduction of at least 30% of whole-body mass in a subject. In another preferred embodiment, weight loss is a reduction of at least 40% of whole-body mass in a subject.

In some preferred embodiments, the pharmaceutical composition for use according to the invention is for use in treating age-related kyphosis. As defined herein, the term "kyphosis" refers to a condition of the thoracic region of the spinal column where a dorsally exaggerated curvature is observed, possibly due to age-related reduction in muscle mass or due to osteoporosis. In one embodiment, kyphosis is characterized by a rounded upper back, or in extreme cases, a 'hump-back'. In another preferred embodiment, kyphosis is attributed to weakness of the spinal extensor musculature, wherein these muscles include the erector spinae (iliocostalis, longissimus and spinalis), thoracis, interspinales and the multifidus.

In another embodiment, as would be apparent to one of ordinary skill in the art, assessment of thoracic kyphosis is performed by standing lateral spine radiographs. In some embodiments, spinal radiographs may be taken in the supine position for comfort. The Cobb's angle of kyphosis is calculated from perpendicular lines drawn on a standard thoracic spine radiograph: a line extends through the superior endplate of the vertebral body, marking the beginning of the thoracic curve (usually at T4), and the inferior endplate of the vertebral body, marking the end of the thoracic curve (usually at T12). In another embodiment, as would be apparent to one of ordinary skill in the art, acceptable alternatives for assessment of thoracic kyphosis include, but are not limited to, the Debrunner kyphometer and the flexicurve ruler, both performed standing. In another embodiment, kyphosis index is calculated as the width divided by the length of the thoracic curve, multiplied by 100. In another embodiment, a kyphosis index value greater than 13 defines hyperkyphosis. In another embodiment, the terms "kyphosis" and "hyperkyphosis" are used herein interchangeably.

In some preferred embodiments, the pharmaceutical compositions for use according to the invention are for use in treating age-related pancreatic steatosis. As used herein, "pancreatic steatosis" refers to the accumulation of fat in the pancreatic gland (i.e., the pancreas). Pancreatic steatosis may also be known as: pancreatic lipomatosis, fatty replacement, fatty infiltration, fatty pancreas, lipomatous pseudohypertrophy and non-alcoholic fatty pancreatic disease, among others.

In one embodiment, pancreatic steatosis may be assessed by an imaging method. Non-limiting examples of a method applicable in assessing pancreatic steatosis include, but are not limited to, standard histology, ultrasonography, computed tomography (CT) and magnetic resonance imaging (MRI), among others.

As would be apparent to one of ordinary skill in the art, at least three methods are capable at quantifying pancreatic fat accumulation using MRI: (a) frequency shift between fat and water resonances; (b) the Dixon method; and (c) spectral-spatial excitation technique.

As used herein, the terms "acute pancreatitis" refers to inflammation of the pancreas that occurs when digestive enzymes leak out of their collecting ducts and damage the surrounding tissue. In one embodiment, in acute pancreatitis digestive enzymes are released in their activated form from the exocrine portions of the pancreas, thereby causing inflammation, injury, autolysis and necrosis to the organ (i.e., the pancreas). In another embodiment, acute pancreatitis results in hemorrhage and pseudocyst formation within the gland. Common symptoms of acute pancreatitis include, but are not limited to, severe upper abdominal pain, nausea and vomiting.

In some preferred embodiments, the pharmaceutical compositions for use according to the invention are for use in treating age-related hepatosteatosis. As defined herein, the term "age-related fatty liver disease (FLD)" refers to a liver condition that occurs when lipids accumulate in hepatocytes (i.e. liver cells) and further impair hepatic microvascular circulation. In one example described herein not falling within the scope of the claims, FLD can progress to more advanced liver disease such as nonalcoholic steatohepatitis (NASH; metabolic steatohepatitis). In one example described herein not falling within the scope of the claims, NASH may progress to further liver damage ultimately leading to chronic liver failure and, in some cases, hepatocellular carcinoma.

Fatty liver diseases can be diagnosed in a subject by multiple methods. As would be apparent to one of ordinary skill in the art, methods for diagnosing FLD include, but are not limited to, physical examination, blood test, imaging, tissue biopsy, or a combination thereof. In one example described herein not falling within the scope of the claims, physical examination for detecting fatty liver in a subject includes seeking for an enlarged liver. In another example described herein not falling within the scope of the claims, physical examination for detecting fatty liver in a subject includes examination of the subject's medical history of alcohol use, medication use, supplement use, or a combination thereof. In one example described herein not falling within the scope of the claims, blood test for detecting fatty liver in a subject includes measuring the concentrations of liver enzymes, such as, but not restricted to, aspartate transaminase (AST), and alanine transaminase (ALT). In another example described herein not falling within the scope of the claims, concentration of liver enzymes may be represented by the calculated ratio of AST to ALT, as would be apparent to one of ordinary skill in the art. In one example described herein not falling within the scope of the claims, imaging methods for detecting fatty liver in a subject include any one of ultrasound, computational tomography (CT), or magnetic resonance imaging (MRI). In one example described herein not falling within the scope of the claims, detecting fatty liver in a subject comprises collection of a tissue biopsy. In another example described herein not falling within the scope of the claims, detecting fatty liver in a subject's tissue biopsy comprises sectioning, staining, or both. In another example described herein not falling within the scope of the claims, one skilled in the art will appreciate that specific markers may be employed for the detection of specifically expressed genes and products thereof, instead of a general chemical stain (i.e., acidophilic stain, basophilic stain, charge-based stain, and the like).

In the pharmaceutical composition for use according to the invention, at least one age-related symptom or disease is selected for treatment from whole body weight loss of the elderly, kyphosis, pancreatic steatosis and hepatosteatosis.

As used herein, the terms "treatment" or "treating" of a disease, disorder, or condition encompasses alleviation of at least one symptom thereof, a reduction in the severity thereof, or inhibition or slowing of the progression thereof. Treatment need not mean that the disease, disorder, or condition is totally cured. To be an effective treatment, a useful composition herein needs only to reduce the severity of a disease, disorder, or condition, reduce the severity of symptoms associated therewith, or provide improvement to a patient or subject's quality of life.

As used herein, the term "prevention" of a disease, disorder, or condition encompasses the delay, prevention, suppression, or inhibition of the onset of a disease, disorder, or condition. As used in accordance with the presently described subject matter, the term "prevention" relates to a process of prophylaxis in which a subject is exposed to the presently described peptides prior to the induction or onset of the disease/disorder process. This could be done where an individual has a genetic pedigree indicating a predisposition toward occurrence of the disease/disorder to be prevented. For example, this might be true of an individual whose ancestors show a predisposition toward certain types of, for example, inflammatory disorders. The term "suppression" is used to describe a condition wherein the disease/disorder process has already begun but obvious symptoms of the condition have yet to be realized. Thus, the cells of an individual may have the disease/disorder, but no outside signs of the disease/disorder have yet been clinically recognized. In either case, the term prophylaxis can be applied to encompass both prevention and suppression. Conversely, the term "treatment" refers to the clinical application of active agents to combat an already existing condition whose clinical presentation has already been realized in a patient. In some embodiments, treatment refers to a clinical application of active agents to combat an already existing condition whose clinical presentation has yet to be realized in a patient.

As used herein, the term "condition" includes anatomic and physiological deviations from the normal that constitute an impairment of the normal state of the living animal or one of its parts, that interrupts or modifies the performance of the bodily functions.

Any concentration ranges, percentage range, or ratio range recited herein are to be understood to include concentrations, percentages or ratios of any integer within that range and fractions thereof, such as one tenth and one hundredth of an integer, unless otherwise indicated.

In some preferred embodiments of the pharmaceutical composition for use according to the invention, VEGF-A free or circulating plasma levels are maintained for at least 30 days at a level of 3-fold increased amount compared to a baseline (basal VEGF-A plasma levels in the subject). As used herein, the term "maintain" refers to keeping at a relatively constant level. In some embodiments, "maintain" is keeping a constant level on average across time (e.g. at least one day, at least one week, and at least one month). In one embodiment, a constant level comprises equilibrium. In one embodiment, a constant level comprises a steady state. In some embodiments, maintained levels are fluctuating across time. As used herein, the term "fluctuating" comprises an increase and subsequent decrease, or decrease and subsequent increase, by not more than 0.1%, 0.5%, 1%, 5%, 10%, 20%, 30%, 40%, 50%, 60%, 85% or 90% across time, or any value and range therebetween. Each possibility represents a separate embodiment of the invention. As used herein, fluctuating comprises an increase and subsequent decrease, or decrease and subsequent increase by 0.1-0.5%, 0.4-2%, 1-5%, 4-10%, 9-20%, 15-30%, 25-40%, 30-50%, 40-60%, 55-85%, or 80-100% across time. Each possibility represents a separate embodiment of the invention.

**VEGF, VEGFR and stimulating compounds**

As used herein, VEGF refers to the "vascular endothelial growth factor". In one embodiment, VEGF-A is VEGF-A under accession number NP_001303939.1. In another embodiment, human VEGF-A is comprised of 5 isoforms resulting of alternative splicing of mRNA encoding 121, 145, 165, 189 or 206 amino acids in length ($VEGF_{121-206}$), all of which are capable of stimulating mitogenesis in endothelial cells.

In some embodiments, the present invention further comprise a step of detecting VEGF state in a subject by determining the plasma levels of VEGF in the subject.

In one embodiment, standard VEGF serum level ranges from 1-100 pg/ml. In one embodiment, standard VEGF serum level ranges from 2-250 pg/ml. In one embodiment, standard VEGF serum level ranges from 5-500 pg/ml. In one embodiment, standard VEGF serum level ranges from 5-750 pg/ml. In one embodiment, standard VEGF serum level ranges from 10-1,000 pg/ml. In one embodiment, standard VEGF serum level ranges from 150-1,500 pg/ml. In one embodiment, standard VEGF serum level ranges from 500-2,500 pg/ml. In another embodiment, the term "standard" used herein, is interchangeable with any of "normal", "regular" "proper", "naïve" or "healthy".

In some embodiments, standard VEGF plasma level ranges from 1 to 250 pg/ml. In one embodiment, standard VEGF plasma level ranges from 1-20 pg/ml. In one embodiment, standard VEGF plasma level ranges from 15-25 pg/ml. In one embodiment, standard VEGF plasma level ranges from 20-40 pg/ml. In one embodiment, standard VEGF plasma level ranges from 25-50 pg/ml. In one embodiment, standard VEGF plasma level ranges from 35-75 pg/ml. In one embodiment, standard VEGF plasma level ranges from 50-100 pg/ml. In one embodiment, standard VEGF plasma level ranges from 75-250 pg/ml.

The term "determining" is used in the broadest sense, including qualitative and quantitative determination of the target molecule. In one embodiment, the determining step described herein is only used to identify the presence of VEGF in a biological sample. In another embodiment, the determining step is used to detect levels of VEGF in specimens. In yet another embodiment, the determining step can be used to quantify the amount of VEGF in at least one sample, and further compare VEGF levels between different samples.

In some embodiments, VEGF levels can be determined in a biological sample by any method known to one of ordinary skill in the art, Non-limiting examples for such determination methods include, but are not limited to, immunoassays (e.g., enzyme-linked immunosorbent assay (ELISA), sandwich ELISA, immunohistochemistry, immunocytochemistry, etc.), polymerase chain reaction (PCR) (e.g., quantitative PCR, RT-PCR, etc.), and others.

As used herein, the term "biological sample" refers to any type of physical specimen which has been obtained, collected, derived, dissected or any equivalent thereof, from an animal. In some embodiments, the biological sample comprises biological fluids selected from: serum, plasma, vitreous fluid, lymph fluid, synovial fluid, follicular fluid, seminal fluid, amniotic fluid, milk, whole blood, urine, cerebrospinal fluid, saliva, sputum, tears, perspiration, mucus, or tissue culture media, or a combination thereof. Each possibility represents a separate embodiment of the invention. In some embodiments, the biological sample is selected from: tissue extracts, homogenized tissue, cellular extracts, or a biopsy, or a combination thereof. Each possibility represents a separate embodiment of the invention.

In another embodiment, the biological sample is obtained from a mammal. In another embodiment, the biological sample is obtained from a human.

Methods for obtaining a biological sample from an animal or a subject are common, and would be apparent to one of ordinary skill in the art.

In one embodiment, a vector or a plasmid comprising the VEGF-A encoding sequence may be used. In one embodiment, a vector or a plasmid is a composite vector or plasmid. In one embodiment, a vector or a plasmid is a man-made vector or plasmid comprising at least one DNA sequence which is artificial. In one embodiment, the present invention provides a vector or a plasmid comparing: Adeno Associated Virus, pcDNA3, pcDNA3.1(+/-), pGL3, pZeoSV2(+/-), pSec-Tag2, pDisplay, pEF/myc/cyto, pCMV/myc/cyto, pCR3.1, pSinRep5, DH26S, DHBB, pNMT1, pNMT41, pNMT81, which are available from Invitrogen, pCl which is available from Promega, pMbac, pPbac, pBK-RSV and pBK-CMV which are available from Strategene, pTRES which is available from Clontech, and their derivatives.

In one embodiment, the vector or a plasmid comprises regulatory elements from eukaryotic viruses such as retroviruses. SV40 vectors include pSVT7 and pMT2. In some embodiments, vectors derived from bovine papilloma virus include pBV-1MTHA, and vectors derived from Epstein Bar virus include pHEBO, and p2O5. Other exemplary vectors include pMSG, pAV009/A+, pMTO10/A+, pMAMneo-5, baculovirus pDSVE, and any other vector allowing expression of proteins under the direction of the SV-40 early promoter, SV-40 later promoter, metallothionein promoter, murine mammary tumor virus promoter, Rous sarcoma virus promoter, polyhedrin promoter, or other promoters shown effective for expression in eukaryotic cells.

According to some embodiments, a recombinant adeno-associated vector (AAV) comprising one or more polynucleotide sequence encoding the VEGF-A is provided under the pharmaceutical composition for use according to the present invention.

In one embodiment, various methods can be used to introduce the expression vector into cells. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986] and include, for example, stable or transient transfection, lipofection, electroporation and infection with recombinant viral vectors. In addition, see U.S. Patent Nos. 5,464,764 and 5,487,992 for positive-negative selection methods.

In some embodiments, introduction of nucleic acid by viral infection offers several advantages over other methods such as lipofection and electroporation, since higher transfection efficiency can be obtained due to the infectious nature of viruses.

In one embodiment, it will be appreciated that the polypeptides encoded by the nucleic acid of the pharmaceutical composition for use according to the present invention can also be expressed from a nucleic acid construct administered to the individual employing any suitable mode of administration, described hereinabove (i.e., *in-vivo* gene therapy). In one embodiment, the nucleic acid construct is introduced into a suitable cell via an appropriate gene delivery vehicle/method (transfection, transduction, homologous recombination, etc.) and an expression system as needed and then the modified cells are expanded in culture and returned to the individual (i.e., *ex-vivo* gene therapy).

In some embodiments, the pharmaceutical compositions comprise solutions or emulsions, which in some embodiments are aqueous solutions or emulsions comprising a safe and effective amount of the compounds of the pharmaceutical compositions for use according to the present invention and optionally, other compounds, intended for topical intranasal administration. In some embodiments, the pharmaceutical compositions comprise from about 0.01% to about 10.0% w/v of a subject compound, more preferably from about 0.1% to about 2.0, which is used for systemic delivery of the compounds by the intranasal route.

In some embodiments, the pharmaceutical compositions for use according to the invention further comprise an acceptable carrier or diluent. In some embodiments, the carrier or diluent is a pharmaceutically acceptable carrier or diluent.

In another embodiment, the pharmaceutical compositions for use according to the invention are administered by intravenous, intra-arterial, or intramuscular injection of a liquid preparation. In some embodiments, liquid formulations include solutions, suspensions, dispersions, emulsions, oils and the like. In one embodiment, the pharmaceutical compositions are administered intravenously, and are thus formulated in a form suitable for intravenous administration. In another embodiment, the pharmaceutical compositions are administered intra-arterially, and are thus formulated in a form suitable for intra-arterial administration. In another embodiment, the pharmaceutical compositions are administered intramuscularly, and are thus formulated in a form suitable for intramuscular administration.

In another embodiment, the pharmaceutical compositions are applied topically to body surfaces, and are thus formulated in a form suitable for topical administration or application. Suitable topical formulations include gels, ointments, creams, lotions, drops and the like. For topical administration, the compounds of the pharmaceutical compositions for use according to the present invention are combined with an additional appropriate therapeutic agent or agents, prepared and applied as solutions, suspensions, or emulsions in a physiologically acceptable diluent with or without a pharmaceutical carrier.

In one embodiment, the pharmaceutical compositions are manufactured by processes well known in the art, e.g., by means of conventional mixing, dissolving, granulating, dragee-making, levigating, emulsifying, encapsulating, entrapping or lyophilizing processes.

In one embodiment, a pharmaceutical composition for use according to the invention is formulated in conventional manner using one or more physiologically acceptable carriers comprising excipients and auxiliaries, which facilitate processing of the active ingredients into preparations which, can be used pharmaceutically. In one embodiment, formulation is dependent upon the route of administration chosen.

In one embodiment, injectable compositions are formulated in aqueous solutions. In one embodiment, injectable compositions are formulated in physiologically compatible buffers such, but not limited to Hank's solution, Ringer's solution, or physiological salt buffer. In some embodiments, for transmucosal administration, penetrants appropriate to the barrier to be permeated are used in the formulation. Such penetrants are generally known in the art.

In one embodiment, the preparations described herein are formulated for parenteral administration, e.g., by bolus injection or continuous infusion. In some embodiments, formulations for injection are presented in unit dosage form, e.g., in ampoules or in multi-dose containers with optionally, an added preservative. In some embodiments, the compositions comprise suspensions, solutions or emulsions in oily or aqueous vehicles, and comprise formulatory agents such as suspending, stabilizing and/or dispersing agents.

The compositions also comprise, in some embodiments, preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic acid, acetylcysteine, sodium metabisulfite and others; aromatic agents; viscosity adjustors, such as polymers, including cellulose and derivatives thereof; and polyvinyl alcohol and acid and bases to adjust the pH of these aqueous compositions as needed. The compositions also comprise, in some embodiments, local anesthetics or other actives. The compositions can be used as sprays, mists, drops, and the like.

In some embodiments, pharmaceutical compositions for parenteral administration comprise aqueous solutions of the active preparation in water-soluble form. Additionally, suspensions of the active ingredients, in some embodiments, are prepared as appropriate oily or water-based injection suspensions. Suitable lipophilic solvents or vehicles include, in some embodiments, fatty oils such as sesame oil, or synthetic fatty acid esters such as ethyl oleate, triglycerides or liposomes. Aqueous injection suspensions contain, in some embodiments, substances which increase the viscosity of the suspension, such as sodium carboxymethyl cellulose, sorbitol or dextran. In another embodiment, the suspension further comprises suitable stabilizers or agents which increase the solubility of the active ingredients to allow for the preparation of highly concentrated solutions.

In another embodiment, the active compound can be delivered in a vesicle or particularly in a liposome (see Langer, Science 249:1527-1533 (1990); Treat et al., in Liposomes in the Therapy of Infectious Disease and Cancer, Lopez- Berestein and Fidler (eds.), Liss, New York, pp. 353-365 (1989); Lopez-Berestein, ibid., pp. 317-327; see generally ibid).

In another embodiment, the pharmaceutical composition delivered in a controlled release system is formulated for intravenous infusion, implantable osmotic pump, transdermal patch, liposomes, or other modes of administration. In one embodiment, a pump is used (see Langer, supra; Sefton, CRC Crit. Ref. Biomed. Eng. 14:201 (1987); Buchwald et al., (1980); Saudek et al., (1989). In another embodiment, polymeric materials can be used. In yet another embodiment, a controlled release system can be placed in proximity to the therapeutic target, thus requiring only a fraction of the systemic dose (see, e.g., Goodson, in Medical Applications of Controlled Release, supra, vol. 2, pp. 115-138 (1984). Other controlled release systems are discussed in the review by Langer (1990).

In some embodiments, the active ingredient is in a powder form for constitution with a suitable vehicle, e.g., sterile, pyrogen-free water-based solution, before use. Compositions are formulated, in some embodiments, for atomization and inhalation administration. In another embodiment, compositions are contained in a container with attached atomizing means.

In one embodiment, the pharmaceutical composition for use according to the present invention is formulated in rectal compositions such as suppositories or retention enemas, using, e.g., conventional suppository bases such as cocoa butter or other glycerides.

In some embodiments, pharmaceutical compositions for use according to the present invention include compositions wherein the active ingredients are contained in an amount effective to achieve the intended purpose. In some embodiments, a therapeutically effective amount means an amount of active ingredients effective to prevent, alleviate or ameliorate symptoms of disease or prolong the survival of the subject being treated.

In one embodiment, determination of a therapeutically effective amount is well within the capability of those skilled in the art.

In some embodiments, preparation of effective amount or dose can be estimated initially from in vitro assays. In one embodiment, a dose can be formulated in animal models and such information can be used to more accurately determine useful doses in humans.

In one embodiment, toxicity and therapeutic efficacy of the active ingredients described herein can be determined by standard pharmaceutical procedures in vitro, in cell cultures or experimental animals. In one embodiment, the data obtained from these in vitro and cell culture assays and animal studies can be used in formulating a range of dosage for use in human. In one embodiment, the dosages vary depending upon the dosage form employed and the route of administration utilized. In one embodiment, the exact formulation, route of administration and dosage can be chosen by the individual physician in view of the patient's condition. [See e.g., Fingl, et al., (1975)].

In one embodiment, dosing can be of a single or a plurality of administrations, with course of treatment lasting from several days to several weeks or until cure is affected or diminution of the disease state is achieved. In another embodiment, dosing can depend on severity and responsiveness of the condition to be treated.

In one embodiment, the amount of a composition to be administered will, of course, be dependent on the subject being treated, the severity of the affliction, the manner of administration, the judgment of the prescribing physician, etc.

In some embodiment, the term "therapeutically effective amount" refers to a concentration of a VEGF-, VEGFR-stimulating compound, or any combination thereof, effective to treat a disease or disorder in an animal, such as a mammal. The term "a therapeutically effective amount" refers to an amount effective, at dosages and for periods of time necessary, to achieve the desired therapeutic or prophylactic result. The exact dosage form and regimen would be determined by the physician according to the patient's condition.

As used herein, the terms "subject" or "individual" or "animal" or "patient" or "mammal," refers to any subject, particularly a mammalian subject, for whom therapy is desired, for example, a human.

In the discussion unless otherwise stated, adjectives such as "substantially" and "about" modifying a condition or relationship characteristic of a feature or features of an embodiment of the invention, are understood to mean that the condition or characteristic is defined to within tolerances that are acceptable for operation of the embodiment for an application for which it is intended. Unless otherwise indicated, the word "or" in the specification and claims is considered to be the inclusive "or" rather than the exclusive or, and indicates at least one of, or any combination of items it conjoins.

It should be understood that the terms "a" and "an" as used above and elsewhere herein refer to "one or more" of the enumerated components. It will be clear to one of ordinary skill in the art that the use of the singular includes the plural unless specifically stated otherwise. Therefore, the terms "a", "an", and "at least one" are used interchangeably in this application.

For purposes of better understanding the present teachings and in no way limiting the scope of the teachings, unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques.

In the description and claims of the present application, each of the verbs, "comprise", "include", and "have" and conjugates thereof, are used to indicate that the object or objects of the verb are not necessarily a complete listing of components, elements or parts of the subject or subjects of the verb.

Other terms as used herein are meant to be defined by their well-known meanings in the art.

Unless specifically stated or obvious from context, as used herein, the term "or" is understood to be inclusive.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

## EXAMPLES

Generally, the nomenclature used herein, and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds.) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); and Mishell and Shiigi (eds), "Strategies for Protein Purification and Characterization - A Laboratory Course Manual" CSHL Press (1996).

## Materials and Methods

### Mice model

Experiments involving mice were performed according to the Hebrew University guidelines and laws, in compliance with the protocols approved by the Hadassah Medical School animal ethics committees. Transgenic mice expressing a tetracycline-regulated transactivator protein (tTA) mostly in the liver (driver line) were mated with transgenic mice harboring a VEGF164-encoding transgene driven by a tetracycline-responsive promoter (responder line). Pups that inherited both transgenes were selected for modulating VEGF expression, whereas littermates that inherited only the driver transgene served as controls. All mice were kept under tetracycline (160 mg/ml) in sweetened water (2% commercial sugar).

### Levels of circulating VEGF

Once a month, 100 $\mu$l of blood were collected through the tail vein into 10% buffer sodium citrate or K3EDTA. Plasma was prepared by centrifugation (2,060 g for 20 min) and VEGF concentration was measured by Enzyme-linked immunosorbent assay (ELISA) according to manufacturer instructions (R&D Systems).

### Complete blood counts (CBC)

Once in three months, 12 $\mu$l of blood were collected from the tail vein into 12 $\mu$l of heparin solution. CBC was done using an Auto Hematology Analyzer (BC-2800Vet, Mindray).

### Survival curve

Two cohorts of mice, kept under tetracycline in the drinking water, were monthly weighed and monitored for changes in health status. Mice displaying signs of severe altered health were euthanized according to the Authority for Biological and Biomedical model's policy at the Hebrew University. The survival curve was created using GraphPad prism software.

### Tissue processing for histological analysis

VEGF-treated and control littermates were always sacrificed on the same day. Mice were sacrificed by lethal dose injection of pentobarbital (1 mg/gr mouse). Tissues were harvested, immediately, fixed in 4% buffered formalin and processed for paraffin embedding. Sections of 6 $\mu$m were stained routinely with either Hematoxylin and Eosin or Masson trichrome.

### Rotarod test

Mice were brought to the experimental room 20 min before testing, to ensure they are fully awake. Mice were rested for 20 min by a return to the home cage after each motor test to allow recovery of muscular strength and a return to normal levels of arousal. Mice were placed on the rotating rod, facing away from the direction of rotation so they had to walk forward to stay upright. The start speed was adjusted to 4 rpm and the acceleration rate to 20 rpm/min. Maximum speed was 40 rpm. The time at which the mouse fell was recorded. Each mouse was tested in three trials and mean time was calculated.

### Preparation of mouse skeletal tissue for high-resolution 3D confocal imaging

Bones were collected and immediately fixed in 2% buffered fresh paraformaldehyde for 12 hours. Decalcification was done for 48 hr using a solution of 0.5 M EDTA, pH 7.4 at 4 °C with constant agitation. The bone samples are cryoprotected in a 20% (w/v) sucrose and 2% (w/v) polyvinylpyrrolidone (PVP) before subjecting them to embedding and freezing in a solution of 8% (w/v) gelatin with 20% (w/v) sucrose and 2% (w/v) PVP. For the purposes of 3D imaging, 70-150 $\mu$m thick sections were used. For immunohistochemistry, bone sections were permeabilized with a 0.3% (v/v) Triton X-100 solution. To achieve efficient penetration of antibodies into very thick sections (<100 $\mu$m), the inventors prepared the primary antibody solution in 0.05% (v/v) Triton X-100 and incubated the sections for 12 hr at 4 °C. After extensive washing, the sections were incubated with a fluorescent secondary antibody. Imaging was done using a confocal laser-scanning microscopy, using the z-stack scanning to obtain sequential depth imaging of thick bone sections. Three dimensional (3D) reconstructions of images were done using Imaris software.

*Progression of Kyphosis*

Mice were sedated with 50 mg/kg ketamine HCl (Ketamil, Troy Laboratories) in combination with 10 mg/kg xylazine HCl (Ilium Xylazil-20, Troy Laboratories) administered by subcutaneous injection. Mice were lightly taped to the table support. Imaging was done by X-ray radiography (GE OEC 9900 Elite - 52 kVp, 4.30 mA). Kyphosis index (KI) was measured as follows:

a) a line was drawn from posterior edge of C7 (A) to the posterior edge of L6 (B), and termed AB line;
b) a line was drawn from the dorsal border of the vertebral body farthest from the AB line, and termed CD line;

$$(KI) = \frac{AB\ Line}{CD\ Line}$$

c) Kyphosis index

**EXAMPLE 1**

**VEGF overexpression increases life-span and prevents cognitive and tissue deterioration**

VEGF over-expressing mice were found to have approximately 3-fold at most higher circulating VEGF levels (**Fig. 1**). The inventors showed that such individuals over-expressing VEGF outlived control littermates by approximately 50% (Fig. 2; median survival 33 months vs 22.7 months for males (**2A**); 30.85 months vs 22.2 months for females (**2B**)). Furthermore, the inventors found that during adulthood VEGF-overexpressing mice gained less weight while at the age of 24 months control individuals lost substantially more weight and were leaner than the VEGF over-expressing counterparts, weighing 27 ± 3 gr and 40 ± 5 gr, respectively (**Fig**. **3**). Then, the inventors tested how cognitive performance is affected with respect to VEGF overexpression. In a ROTAROD test model, individuals over-expressing VEGF showed comparable or better performance at any testing event compared to control (**Fig. 9**). Furthermore, a severer kyphosis was observed in control mice, compared to the VEGF over-expressing mice (**Fig. 10**). After termination, several tissues were harvested for histological observation. Elongated and branched arteries were observed in the diaphysis of VEGF over-expressing mice, indicating bone perfusion (**Fig. 12**). Micro computational tomography (CT) images of cross sections through femoral bones showed control mice lost approximately 22% more bone tissue than their VEGF littermates (**Fig. 11**).

VEGF over-expressing mice were found to have a richer layer of adipose tissue compared to control mice skin, which was found to accommodate a very few adipocytes (**Fig. 8**). With respect to metabolic tissues, the pancreas, intestine and liver were examined (**Figs. 13, 14 and 7,** respectively). A significant reduction in white adipose tissue (WAT) mass was observed in VEGF mice compared to their control littermates (**Fig. 5A**), which was accompanied by adequate perfusion maintenance of this tissue (**Fig. 5B**). Control WAT was found to accommodate larger immune cell infiltrates (**5F**-**5G**), compared to WAT obtained from VEGF (**Figs**. **5H**-**5I**). Furthermore, islands of beige adipocytes, which are known to have high thermogenic capacity, were observed only in the VEGF mice (**Fig. 5D**).

VEGF over-expressing mice demonstrated hallmark features of a healthy pancreas, intestines and liver compared to control, in which different steatosis and adenomas were observed. Hepatic damage was further demonstrated by increased circulating enzymes (**Figs. 7E-7F**) and hepatocytic mitochondria rough endoplasmic reticulum morphologies (**Figs. 7G-7H**).

In terms of metabolic activity, VEGF-overexpressing mice were found to have significantly increased food intake (**Fig. 4A**), conserved a higher metabolic flexibility while aging, and showed better glucose tolerance at the age of 18 months (**Fig. 6**).

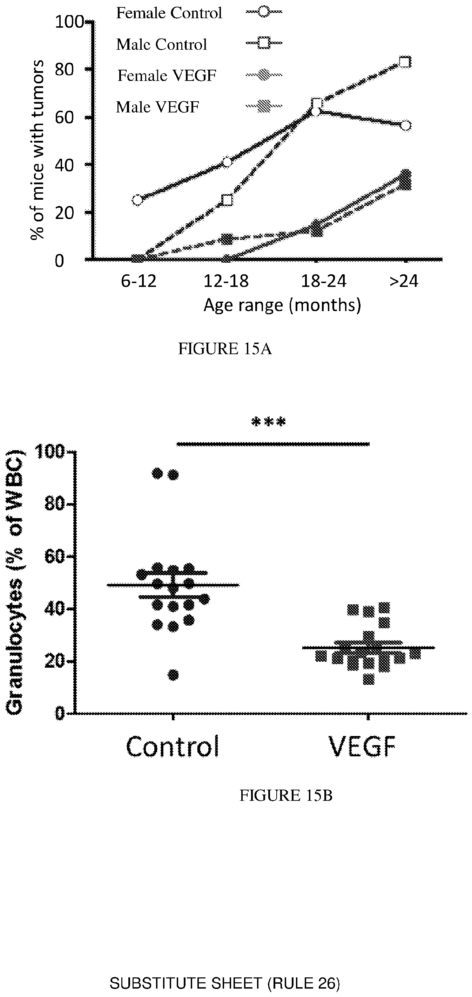

Mice over-expressing VEGF were found to be less prone to spontaneous cancer, as reflected by the percentage of mice presenting at least one spontaneous tumor type at the time of sacrifice. Specifically, in either female or male control mice, neoplastic lesions were observed more often than in the VEGF-overexpressing littermates (**Fig. 15A**). With this respect, a significant increase in circulating granulocytes was observed in the blood of control mice compared to the VEGF-over-expressing littermates (**Fig. 15B**).

Additionally, the inventors showed that levels of circulating soluble VEGF Receptor 1 (VEGFR1, i.e., sFlt1) the increase in aging control mice (**Fig. 16**). During the last months of life, control aged mice comprised significantly higher sFlt1levels compared to control young mice. Therefore, increasing the levels of circulating VEGF, by, for example, inhibiting or blocking sVEGFR, can provide a therapeutic effect with respect to age-related disease, disorder, or symptoms thereof.

**EXAMPLE 2**

**Organ perfusion and neoplasm development**

Vasculature is visualized using *ex-vivo* micro-computed tomography (μCT)-based imaging. Anaesthetized mice are injected with μAngiofil® and after polymerization, organs (including but not limited to brain, heart, thymus, lungs, stomach, kidney, liver, ovary or testis, adrenal, skeletal muscle, abdominal fat) are collected and fixed. Samples are scanned using a desktop microCT and blood vessel sizes are assessed (Matlab) and plotted.

Perfused organs can be further inspected for the presence of neoplastic lesions using immunohistochemistry, immunofluorescence, hematoxylin-eosin staining, and others.

As exemplified herein, mice over-expressing VEGF were found to be less prone to spontaneous cancer. This was reflected by the percentage of mice presenting at least one spontaneous tumor type at the time of sacrifice. Specifically, in either female or male control mice, neoplastic lesions were observed more often than in the VEGF-overexpressing littermates (**Fig**. **15A**).

## EXAMPLE 3

### Plasma levels of hormones and growth factors

Parathyroid hormone (PTH), Follicular stimulating hormone (FSH), Growth hormone (GH), Insulin-like growth factor 1 (IGF-1), Growth Differentiation Factor 11 (GDF11), Myostatin and Estrogen are quantified by means of ELISA (R&D Systems).

## EXAMPLE 4

### Bone density analysis

Bone parameters were evaluated by bone mineral density using $\mu$CT and mechanical testing of the tibia and showed that control mice lost approximately 22% more bone tissue than their VEGF littermates (**Fig. 11**).

## EXAMPLE 5

### Cognitive performance analysis

Cognitive performance is evaluated by watermaze, fear conditioning assay, open-field and novelty recognition assay, and others.

As exemplified herein, the inventors showed that improved cognitive performance correlated with VEGF overexpression. Specifically, using a ROTAROD test model, the inventors had shown that individuals over-expressing VEGF had comparable or better performance at any testing event compared to control (**Fig. 9**).

## EXAMPLE 6

### Fertility

Litter size and number are recorded during 6 months for mice of different ages. Ovaries are collected and processed for immunohistochemistry. Morphological assessment of aging is done according to the number of follicles and corpus luteum as well as atretic follicles.

## EXAMPLE 7

### Organ regeneration

Skin wound healing - Two full-thickness excisions that include the panniculus carnosus are created on the dorsum, and a 0.5 mm thick silicone splint is placed around the wound. A translucent occlusive dressing is applied, digital images are taken, and micro-calipers are used to measure the wound area daily. Blood perfusion is determined using laser Doppler perfusion imaging. Ten days after wounding, both wounds are excised for histological and gene analyses.

Liver regeneration after partial hepatectomy - partial hepatectomy is done and regeneration is monitored by MRI, histological measurement of hepatocyte and endothelial cells proliferation.

Hematopoietic recovery after acute radiation - mice are exposed to sublethal dose total body radiation. Hematopoietic recovery is monitored by cell blood counts analysis and measurements of bone marrow and spleen cellularity.

Muscle regeneration - muscle injuries are induced by intramuscular injection of $BaCl_2$. Regeneration is measured by morphological and morphometric analysis of the regenerating muscle fibers.

## EXAMPLE 8

### Thymic atrophy

Thymuses are harvested and weighed. A hemocytometer is used to count the numbers of thymocytes (Trypan blue viability test). Single cell suspension is analyzed by FACS for cellularity and immunophenotyping. Each individual cell data is collected and analyzed using CellQuest.

## Claims

1. A pharmaceutical composition comprising a vascular endothelial growth factor (VEGF)-stimulating compound for use in a method of treating age-related symptoms or disease selected from whole body weight loss of the elderly, kyphosis, pancreatic steatosis and hepatosteatosis,

   wherein the VEGF-stimulating compound is a nucleic acid comprising a VEGF-A encoding sequence,
   wherein the method comprises administering a therapeutically effective amount of the pharmaceutical composition to a subject in need thereof.

2. The pharmaceutical composition for use according to claim 1, wherein the pharmaceutical composition is administered in an amount effective to increase the amount of free or circulating VEGF-A by 3-fold at most in a subject's plasma compared to a baseline, wherein the baseline is basal VEGF-A plasma levels in the subject.

3. The pharmaceutical composition for use according to claim 2, wherein 3-fold increased amount of free or circulating VEGF-A compared to the baseline is maintained for at least 30 days.

4. The pharmaceutical composition for use according to claim 1, wherein the pharmaceutical composition is administered at least once a month.

5. The pharmaceutical composition for use according to claim 4, wherein the pharmaceutical composition is administered once a month.

**Patentansprüche**

1. Pharmazeutische Zusammensetzung, umfassend eine vaskuläre endotheliale Wachstumsfaktor (VEGF)-stimulierende Verbindung zur Verwendung in einem Verfahren zur Behandlung von altersbedingten Symptomen oder Krankheiten, ausgewählt aus Ganzkörpergewichtsverlust älterer Menschen, Kyphose, Pankreas-Steatose und Hepatosteatose,

   wobei die VEGF-stimulierende Verbindung eine Nukleinsäure ist, umfassend eine VEGF-A codierende Sequenz,
   wobei das Verfahren Verabreichen einer therapeutisch wirksamen Menge der pharmazeutischen Zusammensetzung an ein Individuum umfasst, welches diese benötigt.

2. Pharmazeutische Zusammensetzung zur Verwendung nach Anspruch 1, wobei die pharmazeutische Zusammensetzung in einer Menge verabreicht wird, welche wirksam ist, um die Menge an freiem oder zirkulierendem VEGF-A um höchstens das Dreifache im Plasma eines Individuums im Vergleich zu einem Referenzwert zu erhöhen, wobei der Referenzwert basale VEGF-A-Plasmaspiegel in dem Individuum ist.

3. Pharmazeutische Zusammensetzung zur Verwendung nach Anspruch 2, wobei die dreifach erhöhte Menge von freiem oder zirkulierendem VEGF-A im Vergleich zu dem Referenzwert für mindestens 30 Tage aufrechterhalten wird.

4. Pharmazeutische Zusammensetzung zur Verwendung nach Anspruch 1, wobei die pharmazeutische Zusammensetzung mindestens einmal im Monat verabreicht wird.

5. Pharmazeutische Zusammensetzung zur Verwendung nach Anspruch 4, wobei die pharmazeutische Zusammensetzung einmal im Monat verabreicht wird.

**Revendications**

1. Composition pharmaceutique comprenant un composé stimulant le facteur de croissance endothélial vasculaire (VEGF) à utiliser dans un procédé de traitement de symptômes ou de maladies liés à l'âge sélectionnés parmi la perte de poids corporel des personnes âgées, la cyphose, la stéatose pancréatique et l'hépatostéatose,

   dans laquelle le composé stimulant le VEGF est un acide nucléique comprenant une séquence codant pour le VEGF-A,
   dans laquelle le procédé comprend l'administration d'une quantité thérapeutiquement efficace de la composition pharmaceutique à un sujet qui en a besoin.

2. Composition pharmaceutique à utiliser selon la revendication 1, dans laquelle la composition pharmaceutique est administrée en une quantité efficace pour augmenter la quantité de VEGF-A libre ou circulant d'au plus 3 fois dans le plasma d'un sujet par comparaison à une référence, dans laquelle la référence est des niveaux de plasma de VEGF-A de base chez le sujet.

3. Composition pharmaceutique à utiliser selon la revendication 2, dans laquelle une quantité 3 fois supérieure de VEGF-A libre ou circulant par comparaison à la référence est maintenue pendant au moins 30 jours.

4. Composition pharmaceutique à utiliser selon la revendication 1, dans laquelle la composition pharmaceutique est administrée au moins une fois par mois.

5. Composition pharmaceutique à utiliser selon la revendication 4, dans laquelle la composition pharmaceutique est administrée une fois par mois.

FIGURE 1

FIGURE 2A

FIGURE 2B

FIGURE 3A

FIGURE 3B

FIGURE 3C

FIGURE 4A

FIGURE 4B

FIGURE 4C

FIGURE 4D

FIGURE 4E

FIGURE 4G

FIGURE 4F

FIGURE 5A

FIGURE 5B

FIGURE 5C

FIGURE 5D

FIGURE 5E

FIGURE 5F

FIGURE 5G

FIGURE 5H

FIGURE 5I

FIGURE 6

FIGURE 7A

FIGURE 7C

FIGURE 7B

FIGURE 7D

FIGURE 7E

FIGURE 7F

FIGURE 7G

FIGURE 7H

FIGURE 8A

FIGURE 8C

FIGURE 8B

FIGURE 8D

FIGURE 9

FIGURE 10A

FIGURE 10B

FIGURE 10C

FIGURE 11A          FIGURE 11C

FIGURE 11B          FIGURE 11D

FIGURE 12A          FIGURE 12B          FIGURE 12C

FIGURE 12D          FIGURE 12E          FIGURE 12F

FIGURE 13A

FIGURE 13C

FIGURE 13B

FIGURE 13D

FIGURE 14A

FIGURE 14C

FIGURE 14B

FIGURE 14D

FIGURE 15A

FIGURE 15B

FIGURE 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62656471
- CN 107661492
- WO 200176620 A
- EP 3011957 A
- WO 2016092453 A
- EP 2714063 A
- WO 2007007362 A
- WO 2014020149 A

- US 5464764 A
- US 5487992 A
- US 4666828 A
- US 4683202 A
- US 4801531 A
- US 5192659 A
- US 5272057 A

### Non-patent literature cited in the description

- **JIN BO TANG et al.** Basic FGF or VEGF gene therapy corrects insufficiency in the intrinsic healing capacity of tendons. *Scientific Reports*, 11 February 2016, vol. 6, 1-12
- **BRIDGET M DEASY et al.** Effect of VEGF on the Regenerative Capacity of Muscle Stem Cells in Dystrophic Skeletal Muscle. *Molecular Therapy*, 01 October 2009, vol. 17, 1788-1798
- **AZZOUZ MIMOUN et al.** VEGF delivery with retrogradely transported lentivector prolongs survival in a mouse ALS model. *NATURE*, 27 May 2004, vol. 429, 413-417
- **ROGERS R S et al.** Intracavernosal vascular endothelial growth factor (VEGF) injection and adeno-associated virus-mediated VEGF gene therapy prevent and reverse venogenic erectile dysfunction in rats. *INTERNATIONAL JOURNAL OF IMPOTENCE RESEARCH*, 01 February 2003, vol. 15, 26-37
- **HILTUNEN MIKKO OLAVI et al.** Adenovirusmediated VEGF-A gene transfer induces bone formation in vivo. *THE FASEB JOURNAL, FEDERATION OF AMERICAN SOCIETIES FOR EXPERIMENTAL BIOLOGY*, 01 June 2003, vol. 17 (9), 1-15
- **SAMBROOK et al.** Molecular Cloning: A Laboratory Manual. Cold Springs Harbor Laboratory, 1989
- **AUSUBEL et al.** Current Protocols in Molecular Biology. John Wiley and Sons, 1989
- **CHANG et al.** Somatic Gene Therapy. CRC Press, 1995
- **VEGA et al.** Gene Targeting. CRC Press, 1995

- Vectors: A Survey of Molecular Cloning Vectors and Their Uses. Butterworths, 1988
- **GILBOA**. *Biotechniques*, 1986, vol. 4 (6), 504-512
- **LANGER**. *Science*, 1990, vol. 249, 1527-1533
- Liposomes. **TREAT et al.** Therapy of Infectious Disease and Cancer. Liss, 1989, 353-365
- **SEFTON**. CRC Crit. Ref. Biomed. Eng.. 1987, vol. 14, 201
- **GOODSON**. *Medical Applications of Controlled Release*, 1984, vol. 2, 115-138
- **SAMBROOK et al.** Molecular Cloning: A laboratory Manual. 1989
- Current Protocols in Molecular Biology. 1994, vol. I-III
- **PERBAL**. A Practical Guide to Molecular Cloning. John Wiley & Sons, 1988
- **WATSON et al.** Recombinant DNA. Scientific American Books
- Genome Analysis: A Laboratory Manual Series. Cold Spring Harbor Laboratory Press, 1998, vol. 1-4
- Cell Biology: A Laboratory Handbook. 1994, vol. I-III
- **FRESHNEY**. Culture of Animal Cells - A Manual of Basic Technique. Wiley-Liss, 1994
- Current Protocols in Immunology. 1994, vol. I-III
- Basic and Clinical Immunology. Appleton & Lange, 1994
- Strategies for Protein Purification and Characterization - A Laboratory Course Manual. CSHL Press, 1996